# EUROPEAN PATENT APPLICATION

(11) **EP 3 141 594 A2**
(43) Date of publication of application: **15.03.2017**
(21) Application number: 16198334.1
(22) Date of filing: 11.11.2016
(51) Int. Cl.: C12M 1/00

(54) **METHOD FOR SAMPLING FLUID STREAMS FOR MONITORING CONTAMINANTS IN A CONTINUOUS FLOW**

(71) Applicant: Bayer Pharma Aktiengesellschaft, 13353 Berlin (DE)
(72) Inventor: Dr. Schwan, Peter, 51373 Leverkusen (DE); Kistler, Viktorija, 51368 Leverkusen (LT); Lobedann, Dr. Martin, 51069 Köln (DE)
(74) Representative: BIP Patents

(57) **Abstract**

Disclosed herein is a method for monitoring the concentration of at least one kind of contaminant in a fluid stream, comprising the steps of providing at least two unit operations, providing a fluid stream, which passes said at least two unit operations in a flow path, sampling the fluid stream in a predetermined valid manner, determining the contaminant concentration in the sample in order to monitor the contaminant concentration in the fluid stream, wherein the method is carried out under continuous, closed and pathogen-reduced conditions.

## Description

Conventionally, proteins in biotechnological production are purified in batches. This means that the individual production cycles are handled batchwise and discontinuously, with the product being removed as a whole after completion of a production cycle. For a fresh production cycle, a fresh product cycle or batch must then be started. In the same way the individual process steps are handled batchwise with in most cases the intermediate of a batch is processed as a whole from one feed tank to a second tank which is used as the feed tank for the next process step.

For pharmaceutical production, which is strictly regulated, this batchwise production manner requires great expense in time, technology and personnel for example for the preparation of purified and sterilized bioreactors in order to reliably prevent cross-contamination during product replacement in a multipurpose system or between two product batches and to ensure a germ-free product. This applies both to upstream processing (USP), i.e. the production of biological products in fermenters, and to downstream processing (DSP), i.e. purification of the fermentation products. In fermentation in particular, a germ-free environment is essential for successful cultivation. As a rule, the SIP (SIP = Sterilization-In-Place) technique is used for the sterilization of batch or fed batch fermenters. However, reactor downtime due to preparation procedures can lead to high cost in times where the reactor is not available for production.

Thus, continuous processing for the production of therapeutic proteins gains more and more importance and first solutions for realization of truly continuous systems are emerging. Additionally, continuous processes for the production of therapeutic protein allowing the use of single use technology are especially interesting.

In order to use continuous processing for the production of therapeutic proteins according to guidelines and standards such as GMP, contamination e.g. with pathogens has to be reliably avoided just as it is the case in traditional batch-type processes. Moreover, it also has to be demonstrated that said contamination is reliably avoided. In other words, monitoring for possible contaminants is required. In a traditional batch-type production process for therapeutic proteins the microbial load and other contaminants are tested via sampling the complete product batch. For example host cell protein (HCP) concentration after a chromatography step can be an important criterion. Thus after chromatography has been performed a sample is taken, which is deemed to represent the average host cell protein concentration of the complete product batch.

However, it is the hallmark of continuous production processes e.g. for therapeutic proteins that not a complete product batch is passed through a given unit operation before any part of said complete product batch enters the next unit operation. In consequence, there is no time point for taking a sample which automatically represents the average contaminant concentration. Hence, the traditional approach for demonstrating that the concentration of pathogens and/or other contaminants in a given production cycle meets the required criteria is not applicable.

It was therefore an object of the present invention to provide a simple and inexpensive solution for the required demonstration that the concentration of a given contaminant in a continuous flow meets required criteria such as regulatory parameters.

The invention achieves this object by provision of a method for monitoring the concentration of at least one kind of contaminant in a fluid stream comprising the steps of:
- providing at least two unit operations,
- providing a fluid stream, which passes said at least two unit operations in a flow path,
- sampling the fluid stream in a predetermined valid manner,
- determining the contaminant concentration in the sample in order to monitor the contaminant concentration in the fluid stream,
wherein the method is carried out under continuous, closed and pathogen-reduced conditions.

This method for monitoring the concentration of at least one kind of contaminant has the advantage that it allows for a simple and inexpensive solution for demonstrating that the concentration of a contaminant such as a pathogen in a given continuous flow meets criteria e.g. fixed by regulatory authorities, specified by guidelines such as GMP and/or determined during process characterization studies e.g. criteria specific for a given process under specific production conditions.

As used herein the term "predetermined valid manner" refers to the fact that the sampling needs to be carried out reproducibly and the sampling location and/or time point always has to ensure that the sample either represents the average contaminant concentration or a higher than average contaminant concentration in order to allow the conclusion that the fluid stream of a given production process meets criteria e.g. predetermined by regulatory authorities or specified by guidelines such as GMP.

In other words, instead of taking a sample representing the average contaminant concentration in a whole product batch as it is the case under batch-type production conditions, the sample represents the average contaminant concentration up to the highest contaminant concentration of the fluid stream at given predetermined point in the production process and under continuous flow conditions. Thus, if the contaminant concentration in said sample taken in a predetermined valid manner is below an allowed critical value this means that the contaminant concentration of the complete processed fluid stream is below the critical value.

Moreover, the sampling of the fluid stream in a predetermined valid manner ensures that the contaminant concentration of a given specific production process cycle is comparable to other product processes cycles of the same type and run under the same conditions e.g. in the same facility and by the same company.

As used herein the term "continuous" refers to a method for carrying out at least two processing steps and/or unit operations in series in which the outlet fluid stream (fluid flow) of an upstream step is transported to a downstream step. The downstream step begins processing the fluid flow before the upstream step is completed. Accordingly, continuous transport or transfer of a fluid flow from an upstream unit to a downstream unit means that the downstream unit is already in operation before the upstream is shut down, i.e. that two units connected in series simultaneously process the fluid flow that is flowing through them.

As used herein the term "fluid stream" or "fluid flow" refers to a continuous flow of liquid and/or gas.

In a preferred embodiment the product stream or product flow is the cell-free fluid from a heterogeneous cell culture fluid mixture that contains the product, and to the result of any other steps of the process according to the invention, i.e. the product flow after filtration, after chromatography, after viral clearance, after ultrafiltration, after diafiltration, or after further steps of the process according to the invention, wherein these product flows can then show different concentrations and degrees of purity.

In an alternative embodiment of the method for monitoring the concentration of at least one kind of contaminant the fluid stream does not contain a product. This fluid stream may for example be a fluid stream entering a production process.

As used herein, the expression "at least one" means one or more.

It is also to be understood that, as used herein the terms "the," "a," or "an," mean "at least one," are understood to encompass the plural as well as the singular and should not be limited to "only one" unless explicitly indicated to the contrary.

As used herein the term "contaminants" refers to all components including pathogens that represents critical quality attributes and hence have to be monitored in the production of therapeutic proteins.

As used herein the term "pathogen" refers to microorganisms and viruses.

Critical Quality Attributes (CQA) are chemical, physical, biological and microbiological attributes that can be defined, measured, and continually monitored to ensure final product outputs remain within acceptable quality limits.

As used herein the term "unit" or "unit operation" refers to a device that performs one process step in a production process of a biopharmaceutical and biological macromolecular product and to the process which that specific device performs. In other words, in order to provide the final biopharmaceutical and/or biological macromolecular product several units will have to be passed by the fluid stream until the product has the desired characteristics and/or purity.

As used herein the term "modular system" refers to a series of interconnected modules ("units") for carrying out at least two downstream and/or upstream steps in which a fluid stream can be transported. According to the invention, the units are suitable for continuously conducting a step and can be operated with a continuous fluid stream also referred to as fluid flow (and if it comprises a product also referred to as "product flow"). The individual modules of this "modular system" can be interconnected in any combination. Examples of modules within the meaning of the invention are a filtration module, a chromatography module, an ultrafiltration module, a diafiltration module and a dialysis module.

As used herein the term "modular" means that the individual unit operations can be carried out in separate interconnected modules, wherein the modules are preconfigured, germ-reduced, and closed, and can be interconnected in various combinations.

As used herein the term "flow path" refers to any assembly or containment through which the product flows or is in contact with.

As used herein the term "pathogen-reduced" refers to a state of reduced pathogenic count, i.e. a pathogenic count per area or volume unit of close to zero that is achievable by means of a suitable germ-reducing method, wherein this germ-reducing method can be selected from gamma irradiation, beta irradiation, autoclaving, Ethylene Oxide (ETO) treatment, and "Steam-In-Place" (SIP) and/or Heat in Place treatment.

As used herein the term "disposable articles" means that the respective components coming into contact with the fluid stream, particularly equipment, containers, filters, and connecting elements, are suitable for one-time use followed by disposal, wherein these containers can be made of both plastic and metal. Within the scope of the present invention, the term also comprises disposable articles such as those made of steel that are only used once in the process according to the invention and not used again in the process. These disposable articles, for example those made of steel, are then also designated within the scope of the invention as objects "used as disposable articles." Such used disposable articles can then also be designated in the process according to the invention as "disposable" or "single-use" articles ("SU technology"). In this way, the pathogen-reduced status of the process and modular system according to the invention is improved even more.

As used herein the term "closed" means that the method described is operated in such a way that the fluid stream is not exposed to the room environment. Materials, objects, buffers, and the like can be added from outside, wherein, however, this addition takes place in such a way that exposure of the fluid stream to the room environment is avoided.

As used herein the term "closed" refers to both "functionally closed" as well as "closed".

In detail, a closed process system is designed and operated such that the product is never exposed to the surrounding environment. Additions to and draws from closed systems must be performed in a completely closed fashion. Sterile filters may be used to provide effective barriers from contaminants in the environment. The term "functionally closed" refers to a process that may be opened but is "rendered closed" by a cleaning, sanitization and/or sterilization that is appropriate or consistent with the process requirements, whether sterile, aseptic or low bioburden. These systems shall remain closed during production within the system. Examples include process vessels that may be CIP'd and SIP'd between uses. Non-sterile systems such as chromatography or some filtration systems may also be rendered closed in low bioburden operations if appropriate measures are taken during the particular system setup.

Under certain circumstances it might be useful to sample the fluid stream already before it is sampled in the predetermined valid manner. This could, for example, be the case if the first unit operation is an affinity chromatography. In this setting the fluid stream can also be sampled upon elution from the first column.

In one embodiment of the method for monitoring the concentration of at least one kind of contaminant, wherein the at least kind of contaminant is a microbial contaminant and/or a poisonous contaminant and the method further comprises
- providing at least one filter with pore sizes between 0,05 - 2 µm, which separates the at least two unit operations,
- wherein the fluid stream passes said filter with pore sizes between 0,05 - 2 µm as it flows from the one unit operation to the second, and
- wherein sampling the fluid stream in a predetermined valid manner, is achieved via sampling the fluid stream immediately before it passes said filter with pore sizes between 0,05 - 2 µm.

This embodiment has the advantage, that the concentration of the microbial contaminant and/or the poisonous contaminant is highest directly in front of the filter with pore sizes between 0,05 µm - 2 µm. Thus, if the concentration of the microbial contaminant and/or the poisonous contaminant in a sample taken at this sampling point is below an applicable threshold the concentration of said microbial contaminant and/or the poisonous contaminant in the fluid stream has to be below that applicable threshold.

In other words, since the complete fluid stream has to pass filter with pore sizes between 0,05 - 2 µm in order to reach the subsequent unit operation the microbial concentration and the concentration of other contaminants is the highest on the unfiltrate side of the filter. Hence, in this case the sample no longer represents the average contaminant concentration as it is the case in a batch process, but rather represents the highest contaminant concentration collected over certain period of time. Thus, if the contaminant concentration in said sample is below an applicable threshold the contaminant concentration of the complete processed fluid stream is below the applicable threshold.

The step of determining the concentration of microbial contaminants can be carried out e.g. when the filter is exchanged or in predetermined intervals or when a given characteristic of the fluid stream or the filter has reached a predetermined threshold.

As used herein the term "unfiltrate" refers to the substance that is retained by a given filter. In other words, while the filtrate passes the filter the unfiltrate remains in or before the filter.

As used herein the term "poison" or "poisonous contaminant" refers to all components, which are potentially harmful to humans, animals and plants e.g. via a chemical reaction or other activity on the molecular scale, when an organism absorbs a sufficient quantity.

As used herein the term "toxin" refers to small molecules, peptides, or proteins that are capable of causing disease on contact with or absorption by body tissues interacting with biological macromolecules such as enzymes or cellular receptors such as bacterial endotoxins, bacterial exotoxins and fungal biotoxins. In other words a toxin is a type of poisonous substance produced within living cells or organisms;

As stated above the filter has pores with sizes between 0,05 µm - 2µm, preferably between 0,05 - 0,6 µm, most preferably between 0,1 - 0,2 µm in order to filter the fluid stream and *inter alia* to filter out particles such as aggregated product particles. As used herein the expression "pore sizes between 0,05 µm - 2 µm" refers to the fact that in a given filter the majority of pores has a given size and said given sizes is between 0,05 µm - 2 µm, e.g. the majority of pores has a size of 0,2 µm.

In preferred embodiment the sampling of the fluid stream is carried out before it passes the filter with pore sizes between 0,05 µm - 2µm and said sampling takes places directly in front of the filter or in the venting outlet of the filter.

In another preferred embodiment of the method for monitoring the concentration of contaminants the at least one filter with pore sizes between 0,05 µm - 2 µm is a Sartopore 2 XLG size 8 0.2µm (Sartorius, 5445307G8G).

In a further preferred embodiment of the method for monitoring the concentration of contaminants a multi-port and/or a sterile bag is connected to the unfiltrate side of the filter with pore sizes between 0,05 - 2 µm and said sterile bag can be connected in a closed or functionally closed way in order to take the sample.

In a preferred embodiment the above described method for monitoring the concentration of at least one kind of contaminant, at least two filters with pore sizes between 0,05 µm - 2µm, µm are provided in parallel, so that the first filter can be changed under germ-reduced conditions while the fluid stream passes through the second filter.

In another embodiment of the above described method for monitoring the concentration of at least one kind of contaminant, the sampling of the fluid stream in a predetermined valid manner is achieved via sampling the fluid stream at a predetermined time point in relation to the first and/or the second unit operation and/or sampling the fluid stream when a given characteristic of the fluid stream has reached a predetermined threshold.

This embodiment has the advantage that enables sampling in a "predetermined valid manner" at points - i.e. locations and/or times points - during the production process which do not necessarily comprise a filter.

Moreover, in cases where filtered material is analyzed - e.g. in a setting where the fluid stream is filtered before it enters the first unit operation - this embodiment allows the analysis of filtered material. This is advantageous since the device for analyzing the sample taken in a predetermined valid manner only has to be able to analyze filtered material and not unfiltered material which could potentially block the analysis device due to the presence of larger (unfiltered) particles.

It should be noted that the different embodiments described herein can be combined in any suitable fashion. Thus, one and the same production process may comprises for instance one or more sampling(s) in a predetermined valid manner immediately before a filter with pore sizes between 0,05 µm - 2 µm as well as one or more sampling(s) in predetermined valid manner achieved via sampling the fluid stream at a predetermined time point in relation to the first and/or the second unit operation and/or sampling the fluid stream when a given characteristic of the fluid stream has reached a predetermined threshold. Thus, in theory the sampling points could be located immediately before and after the filter with pore sizes between 0,05 µm - 2 µm.

As used herein the term "flow-through" refers to an operation mode of a chromatographic unit, in which the impurities either specifically bind to the separation medium while the product of interest does not, thus allowing the recovery of the desired product in the "flow-through" and/or in which both the product of interest and one or more impurities bind to the separation medium. In the second case the impurities bind more tightly to the separation medium than the product of interest and hence as loading continues unbound product of interest can be recovered in the "flow through". In other words, the fluid stream leaving the chromatographic unit operation during the entire time when product is loaded on the chromatographic unit operation constitutes the product stream.

As used herein the term "bind and elute" refers to an operation mode of a chromatographic unit, in which the product differentially binds to the chromatographic medium. Hence, a bind and elute type chromatography comprises at least the steps of loading, washing, elution and regeneration of a chromatography column, wherein the fluid stream leaving the chromatography column during elution represents the product stream.

An example of a method for monitoring the concentration of at least one kind of contaminant, wherein the sampling of the fluid stream in a predetermined valid manner is achieved via sampling the fluid stream at a predetermined time point in relation to the first and/or the second unit operation and/or sampling the fluid stream when a given characteristic of the fluid stream has reached a predetermined threshold is sampling a flow-through chromatography column after a product cycle has passed through the column. Without wishing to being bound by theory it was surprisingly found that the sample either represents the average contaminant concentration or a higher than average contaminant concentration in order to allow the conclusion that the fluid stream of a given production process meets criteria e.g. predetermined by regulatory authorities or specified by guidelines such as GMP. Thus, if the contaminant concentration in said sample taken in a predetermined valid manner is below a required critical value this means that the contaminant concentration of the complete processed fluid stream is below the critical value.

The time point for taking the sample in a valid manner can be predetermined in different ways.

The time point can set based on values obtained during experiments for process characterization. For example, in case of an ion exchange chromatography operated in flow through mode it was predetermined that the fluid stream passes the chromatography in two hours. Thus, the time point for taking a sample in a predetermined valid manner is set at 1 hour and 55 minutes.

Likewise the given characteristic can be predetermined in different ways.

One example of sampling the fluid stream when a given characteristic of the fluid stream has reached a predetermined threshold as e.g. a specific product quantity such as an antibody load. For example, in case of a chromatography operated in flow through mode it was predetermined that the maximal column load is 2 g of antibody per liter of column . Thus, when a new column is installed a counter - for instance integrated in an automatic process control system - is set to start and then monitors the column load e.g. via monitoring the flow rate of the fluid stream and the product concentration in the fluid stream for example via 280 nm measurement. As soon as the column load has reached a threshold of 1,95 g of antibody per liter of column a sample is taken.

Another example of sampling the fluid stream when a given characteristic of the fluid stream has reached a predetermined threshold is e.g. when a specific predetermined volume was loaded on a chromatography column. For instance it was predetermined that the critical volume is 2 liters per ml of column. Thus when a column is connected to a flow path a counter - for instance integrated in an automatic process control system - is set to start and then monitors the volume of the fluid stream passing said chromatography column e.g. via monitoring the pump rate of the specific pumps. As soon as the volume has reached a threshold of 1,95 liters per ml of column of passed fluid stream a sample is taken.

Another example of sampling the fluid stream when a given characteristic of the fluid stream has reached a predetermined threshold is e.g. when a specific predetermined volume was eluted from a column in a bind and elute chromatography step. For instance it was predetermined that after a critical elution volume of 2-2.5 column volumes a maximum contaminant concentration is reached. Thus when a column is connected to a flow path a counter - for instance integrated in an automatic process control system - is set to start and and then monitors the elution volume. As soon as the elution volume has reached the threshold closed to the critical elution volume of 2-2.5 column volumes a sample is taken e.g. a differential sample or an integral sample. In case of a differential sample, the sample collection is of a short duration during the period in which the predetermined critical elution volume of 2-2.5 column is reached. In case of an integral sample, the sample collection is continuous during the period in which the predetermined critical elution volume of 2-2.5 column is achieved. Sample collection in an integral fashion can be advantageous if the concentration of several contaminants is to be monitored which are difficult to separate from one another.
In one embodiment of the method for monitoring the concentration of at least one kind of contaminant the fluid stream is product stream.

This product stream for example flows from one unit operation to another unit operation until the product has reached the desired characteristics. This means that so many unit operations may be connected e.g. in a modular fashion as required to reach the desired characteristic of a given product.

The same production process may use both the method for monitoring the concentration of at least one kind of contaminant described herein, wherein the fluid stream is a product stream and the method for monitoring the concentration of at least one kind of contaminant described herein wherein the fluid stream does not contain a product.

In one embodiment of the method for monitoring the concentration of at least one contaminant the product comprises at least one component selected from the group consisting of a peptide, a protein, a small molecule drug, a nucleic acid.

As used herein the term "peptide" refers to a polymer of amino acids of relatively short length (e.g. less than 50 amino acids). The polymer may be linear or branched, it may comprise modified amino acids, and it may be interrupted by non-amino acids. The term also encompasses an amino acid polymer that has been modified; for example, by disulfide bond formation, glycosylation, lipidation, acetylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

As used herein the term "protein" refers to a polypeptide of amino acids. The term encompasses proteins that may be full-length, wild-type, or fragments thereof. The protein may be human, non- human, and an artificial or chemical mimetic of a corresponding naturally occurring amino acid, as well as to naturally occurring amino acid polymers and non- naturally occurring amino acid polymer.

Preferably the protein is a therapeutic protein.

As used herein the term "therapeutic protein" refers to a protein that can be administered to an organism to elicit a biological or medical response of a tissue, an organ or a system of said organism.

Even more preferably the protein is an antibody.

The term "antibody" as used herein refers to a binding molecule such as an immunoglobulin or immunologically active portion of an immunoglobulin, i.e., a molecule that contains an antigen-binding site.

As used herein the term "small molecule drug" refers to a low molecular weight (<900 daltons) compound that may help regulate a biological process.

As used herein, the term "nucleic acid" refers to deoxyribonucleotides or ribonucleotides and polymers thereof in either single- or double-stranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions) and complementary sequences as well as the sequence explicitly indicated.

In a preferred embodiment of the method for monitoring the concentration of at least one kind of contaminant, wherein the sampling of the fluid stream in a predetermined valid manner is achieved via sampling the fluid stream at a predetermined time point in relation to the first and/or the second unit operation and/or sampling the fluid stream when a given characteristic of the fluid stream has reached a predetermined threshold the first of the at least two unit operations through which the fluid stream passes is a bind-and-elute type chromatographic unit operation.

In another preferred embodiment of the method for monitoring the concentration of at least one kind of contaminant, wherein the sampling of the fluid stream in a predetermined valid manner is achieved via sampling the fluid stream at a predetermined time point in relation to the first and/or the second unit operation and/or sampling the fluid stream when a given characteristic of the fluid stream has reached a predetermined threshold the first of the at least two unit operations through which the fluid stream passes is a flow-through type chromatographic unit operation.
This embodiment has the advantage that the predetermined time point for sampling of the fluid stream in a predetermined valid manner can be chosen in relation to the elution-time of the bind-and elute type chromatographic unit operation.

As used herein the term "elution time" refers to the time in which the continuous chromatography elutes a specific column.

In a preferred embodiment of the method for monitoring the concentration of at least one kind of contaminant, wherein the sampling of the fluid stream in a predetermined valid manner is achieved via sampling the fluid stream at a predetermined time point in relation to the first and/or the second unit operation and/or sampling the fluid stream when a given characteristic of the fluid stream has reached a predetermined threshold the given characteristic of the fluid stream is a predetermined antibody load per column volume and/or a predetermined loading volume of a flow-though type chromatography column and/or a predetermined elution volume of a bind-and-elute type chromatography column.

In a preferred embodiment of the above described method for monitoring the concentration of at least one contaminant the method further comprises the step of comparing the contaminant concentration to a predetermined reference value.

This step enables the assessment whether a contaminant concentration is below the predetermined reference value or not.

In a preferred embodiment of the method for monitoring the concentration of at least one kind of contaminant, the method is performed and controlled by an automated process control system, which draws the samples automatically.

It is preferred that in this embodiment at least two filters with pore sizes between 0,05 - 2 µm are provided in parallel, so that the first filter can be automatically changed under germ-reduced conditions, wherein the automatic filter replacement preferably comprises the following steps:
(i) switching of the flow path to the second, i.e. the new filter when a threshold value is exceeded at a pressure sensor on the unfiltrate side, with closure of the flow path, wherein the product in the first, i.e. the used filter is shifted to the filtrate side, preferably by a gas or a liquid, or when a maximum time of the first used filter in the flow path is exceeded, or when a maximum filtrate volume through the first used filter is exceeded,
(ii) venting of the second new filter via an air filter at a venting valve of the new filters, preferably with the product being transported into the new filter by a feed pump, or in a closed bag attached in a germ-reduced manner,
(iii) detection of completion of venting of the second new filter on the unfiltrate side by the pressure sensor or a filling level sensor or a balance or a liquid detector,
(iv) opening of the filtrate outlet and closure of the flow path between the venting valve and air filter via a valve, and
(v) replacement of the old filter with a new filter.
the simultaneous or downstream transport of product into the new filter can be carried out e.g. using a feed pump.

In a preferred embodiment of the method for monitoring the concentration of at least one kind of contaminant, all components coming into contact with the fluid stream are sterilized by means of suitable germ reduction, wherein the germ reduction method is preferably selected from the group composed of gamma irradiation, beta irradiation, autoclaving, ethylene oxide (ETO) treatment, ozone treatment (O₃), hydrogen peroxide treatment (H₂O₂), and steam-in-place (SIP) treatment

In a preferred embodiment of the method for monitoring the concentration of at least one kind of contaminant, the fluid stream is temporarily retained in a storage bag and the fluid stream is transiently mixed in said storage bag prior to sampling the fluid stream in a predetermined valid manner.

Such storage containers are frequently used in continuous processes in order to account for the differences in processing time required by different unit operations. However, a constant mixing in said storage bags can have adverse effects - e.g. shear stress, formation of subvisible particles and/or aggregates - on a product comprised in the fluid stream.

Now it was surprisingly discovered that transient mixing i.e. mixing during a short time interval prior to drawing a sample, of the fluid stream in the storage bag does not have adverse effects on a product comprised in the fluid stream, while at the same time ensuring a homogenous sample, which represents the average composition of the fluid stream.

The short time interval during which the transient mixing takes place preferably has a duration of 30 seconds - 10 min, more preferably between 1 min and 5 min, most preferably between 2 min - 4min in order to minimize potential damage to the product.

Such a transient mixing may be carried out automatically e.g. via a recirculation pump.

In a preferred embodiment of the method for monitoring the concentration of at least one kind of contaminant, all components coming into contact with the fluid stream are disposable articles are or are used as disposable articles.

Moreover, it is possible that different embodiments of the method for monitoring the concentration of at least one kind of contaminant and especially the different ways of sampling the fluid stream in a predetermined valid manner are chosen at different points that a fluid stream passes during a given production process. In other words a combination of the different embodiments of the method for monitoring the concentration of at least one kind of contaminant can be employed in one and the same production process.

In another aspect what is described herein relates to using the method for monitoring the concentration of at least one kind of contaminant in a continuous process for the production of therapeutic proteins.

In a preferred embodiment of said use the method is applied to a process for the continuous, germ-reduced production and/or processing therapeutic protein such as an antibody from a heterogeneous cell culture fluid mixture, comprising the steps:
(a) preparation of a particle-free fluid from a heterogeneous cell culture fluid mixture that contains the product in the form of a product flow,
(b) at least one filtration containing a filtrate,
(c) at least two chromatography steps for cleaning the product,
(d) at least one viral clearance, and
(e) at least one ultrafiltration and/or at least one diafiltration of the product flow of steps (b), (c), and/or (d),
characterized in that the at least two chromatography steps of (c) comprise cleaning by means of at least two chromatography columns and/or membrane adsorbers each.

### EXAMPLE:

### Example 1

The method for the production of biopharmaceutical and biological product to which the method described herein was applied usually comprises at least the following production steps, which are usually connected together as follows:

### B. Downstream

- Cell separation
- Buffer or medium exchange preferably with concentration
- Bioburden reduction preferably with sterile filter
- Capture chromatography
- Virus inactivation
- Neutralization, i.e. pH and conductivity adjustment
- Chromatographic intermediate and fine purification
- pH and conductivity adjustment
- Bioburden reduction e.g. with sterile filter
- Buffer exchange and preferably concentration
- Viral filtration
- Filtration with sterile filter.

Three critical processing steps were chosen as example for microbial testing.
1. Neutralization after virus inactivation
2. pH and conductivity adjustment after the final chromatography step
3. Viral filtration

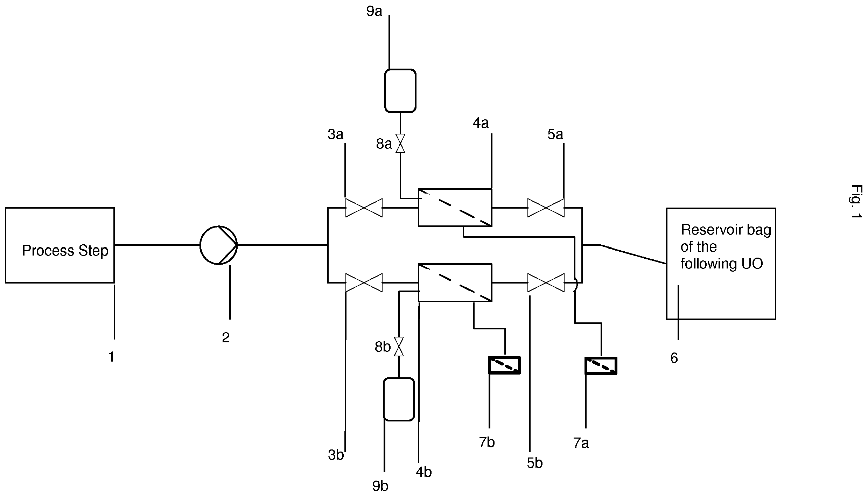

Figure 1 depicts the sampling apparatus that was used at the three sampling spots for microbial and endotoxin sampling, i.e. this is an example of a method for monitoring the concentration of at least one contaminant wherein the at least kind of contaminant is a microbial contaminant and/or a poisonous contaminant. The pump (2) pumps product from the previous process step (1) into the filtration assembly. The product flows only through one active filter either via valve (3a) and (5a) through filter (4a) or via valve (3b) and (5b) through filter (4b) into the storage bag also termed reservoir bag (6) of the subsequent unit operation. As filter a Sartopore 2 XLG size 8 0.2µm (Sartorius, 5445307G8G) was used. These filters are equipped with a hydrophobic 0.2 µm air filter (7a, 7b) for deaeriation at the initial filtration. The flow direction was from Top to Bottom and the air filters were installed on the top vent valve. The bottom vent valve of the filter was equipped with a Cflex tubing (ID 3.2mm ) to which a 1L pre-sterilized flexboys (9a, 9b) could welded on in a closed manner. The samples were taken by opening the pinch valves (8a) or (8b) respectively. For automatic sterile sampling valves (8a) and (8b) - pneumatically or electrically controlled pinch valves - can be used, which can be controlled by a central PCS system.

### Example 2:

### Flowthrough chromatography

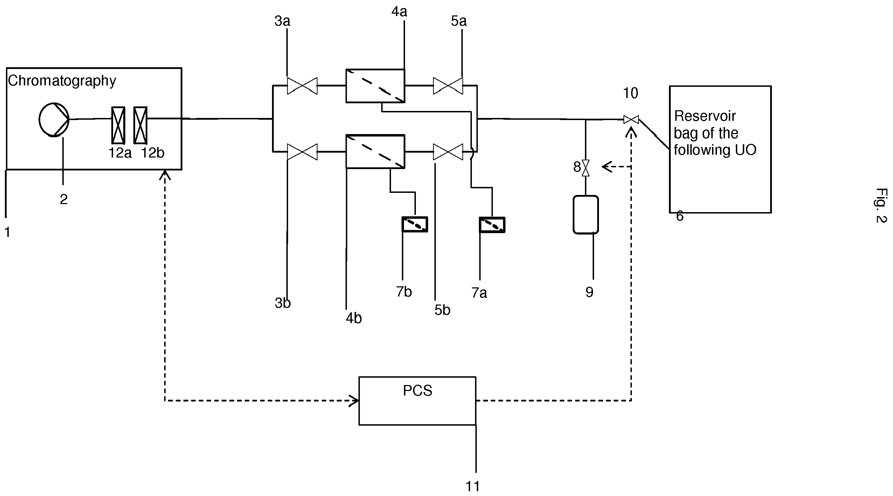

Figure 2 depicts the sampling apparatus that can be used for sampling of non-microbial contaminants after a flow-through chromatography process step, i.e. this is an example of a method for monitoring the concentration of at least one contaminant wherein sampling the fluid stream in a predetermined valid manner is achieved via sampling the fluid stream at a predetermined time point in relation to the first and/or the second unit operation. The load pump (2) of the flow-through chromatography step (1) pumps product either through the chromatography column 12a or column 12b. Both columns use the same resin material and are packed with the same column volume (Vcol). After a certain number of column volumes N the fully loaded chromatography column starts being regenerated, while the second chromatography column is loaded. The flow-through product flows through the filtration assembly into the reservoir bag (6) of the following unit operation. The product flows only through one active filter either via valve 3a and 5a through filter 4a or via valve 3b and 5b through filter 4b into the reservoir bag (6) of the subsequent unit operation. As filter a Sartopore 2 XLG size 8 0.2µm (Sartorius, 5445307G8G) is used in this example. These filters are equipped with a hydrophobic 0.2 µm air filter (7a, 7b) for deaeriation at the initial filtration. The product flows then either via the product valve (10) into the reservoir bag (6) or via the sampling valve (8) into the sampling bag (9). A pre-sterilized sampling bag such as a 1L Flexboy can be used which is installed/ de-installed in a functionally closed or closed manner, such as by sterile tube welding.

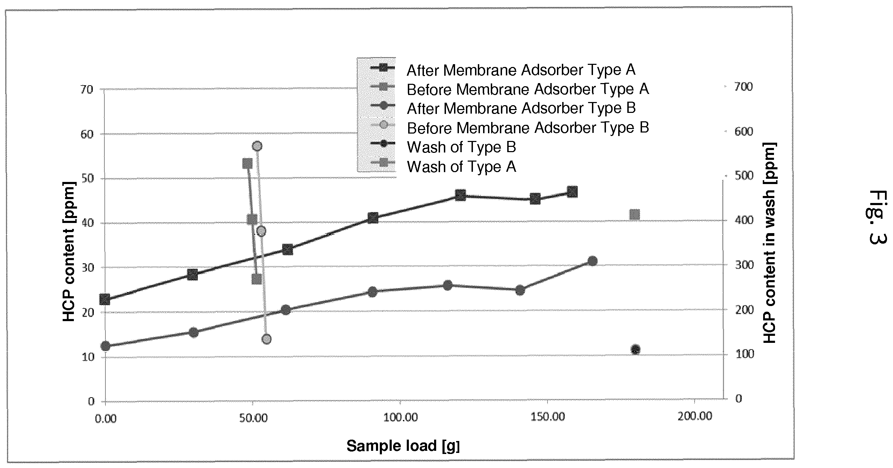

Figure 3 shows that the contaminant concentration of host cell contaminants (HCP) on a membrane adsorber increases with the volume or amount of product loaded onto the chromatography column. If samples were only taken at specific points of time but irrespective of the load onto a chromatography column, the variability in the CQA testing would be high. In order to demonstrate process control of the CQA, the product sample should be taken in the final column volumes of the column loading.

This is achieved by using an automated process control system.

The local control system of the chromatography step (1) integrates the volume applied onto the column in the load phase. If rather the actual amount of protein loaded onto column than the load volume is critical to the CQA in the flowthrough, an online detection method such as UV 280nm can be used. The UV signal is then integrated with the product flowrate. The integrated value is transmitted from the local PCS to the central control system such as a Siemens PCS 7 via for example an OPC or a Profibus protocol. Once the integration value reaches the validated critical sampling threshold i.e. the sampling specification, the sampling routine is started in the central PCS. Thus, sampling in a predetermined valid manner is in this example depended in the predetermined UV signal threshold value. After a possible delay time due to a hold-up in the filtration system, the PCS opens valve 8 and closes valve 9.

Both valves can either be pneumatically or electrically controlled pinch valves.

**The studies which resulted in this application were supported by the grant 031A616M a part of the project "Wissensbasierte Prozessintelligenz-Neue Wege zu stabilen Bioprozessen Teilprojekt M".**

## Claims

1. Method for monitoring the concentration of at least one kind of contaminant in a fluid stream, comprising the steps of:
• providing at least two unit operations,
• providing a fluid stream, which passes said at least two unit operations in a flow path,
• sampling the fluid stream in a predetermined valid manner,
• determining the contaminant concentration in the sample in order to monitor the contaminant concentration in the fluid stream,
wherein the method is carried out under continuous, closed and pathogen-reduced conditions.

2. The method according to claim 1, wherein the at least one kind of contaminant is a microbial contaminant and/or a poisonous contaminant and the method further comprises
• providing at least one filter with pore sizes between 0,05 - 2 µm, which separates the at least two unit operations,
• wherein the fluid stream passes said filter with pore sizes between 0,05 - 2 µm as it flows from the one unit operation to the second, and
• wherein sampling the fluid stream in a predetermined valid manner, is achieved via sampling the fluid stream immediately before it passes said filter with pore sizes between 0,05 - 2 µm.

3. The method according to claim 1 or claim 2, wherein sampling the fluid stream in a predetermined valid manner, is achieved via sampling the fluid stream at a predetermined time point in relation to the first and/or the second unit operation and/or sampling the fluid stream when a given characteristic of the fluid stream has reached a predetermined threshold.

4. The method according to anyone of the preceding claims wherein the fluid stream is a product stream.

5. The method according to claim 3 , wherein the given characteristic of the fluid stream is a predetermined antibody load per column volume and/or a predetermined loading volume of a flow-through type chromatography column and/or a predetermined elution volume of a bind-and-elute type chromatography column.

6. The method according to claim 1, wherein the method further comprises the step of comparing the contaminant concentration to a predetermined reference value.

7. The method according to anyone of the preceding claims wherein the method is performed and controlled by an automated process control system, which draws the samples automatically.

8. The method according to claim 7 wherein at least two filters with pore sizes between 0,05 - 0,2 µm are provided in parallel, so that the first filter can be automatically changed under germ-reduced conditions, wherein the automatic filter replacement preferably comprises the following steps:
(i) switching of the flow path to the second, i.e. the new filter when a threshold value is exceeded at a pressure sensor on the unfiltrate side, with closure of the flow path, wherein the product in the first, i.e. the used filter is shifted to the filtrate side, preferably by a gas or a liquid, or when a maximum time of the first used filter in the flow path is exceeded, or when a maximum filtrate volume through the first used filter is exceeded,
(ii) venting of the second new filter via an air filter at a venting valve of the new filters, preferably with the product being transported into the new filter by a feed pump, or in a closed bag attached in a germ-reduced manner,
(iii) detection of completion of venting of the second new filter on the unfiltrate side by the pressure sensor or a filling level sensor or a balance or a liquid detector,
(iv) opening of the filtrate outlet and closure of the flow path between the bleeder valve and air filter via a valve, and
(v) replacement of the old filter with a new filter.
the simultaneous or downstream transport of product into the new filter can be carried out e.g. using a feed pump.

9. The method according to anyone of the preceding claims, wherein the fluid stream is temporarily retained in a storage bag and the fluid steam is transiently mixed in said storage bag prior to sampling the fluid stream in a predetermined valid manner.

10. The method according to anyone of the preceding claims, wherein all components coming into contact with fluid stream are disposable articles are or are used as disposable articles.

11. Using the method according to any one of the claims 1-10 in a continuous process for the production of a biopharmaceutical, biological, macromolecular product.

12. The method according to claim 11, wherein the method is applied to a process for the continuous, germ-reduced production and/or processing of a biopharmaceutical, biological, macromolecular product from a heterogeneous cell culture fluid mixture, comprising the steps:
(a) preparation of a particle-free fluid from a heterogeneous cell culture fluid mixture that contains the product in the form of a product stream,
(b) at least one filtration containing a filtrate,
(c) at least two chromatography steps for cleaning the product,
(d) at least one viral clearance, and
(e) at least one ultrafiltration and/or at least one diafiltration of the product flow of steps (b), (c), and/or (d),
**characterized in that** the at least two chromatography steps of (c) comprise cleaning by means of at least two chromatography columns and/or membrane adsorbers each.
